# EUROPEAN PATENT SPECIFICATION

(11) **EP 4 061 478 B1**
(45) Date of publication and mention of the grant of the patent: **05.03.2025**
(21) Application number: 20776176.8
(22) Date of filing: 24.09.2020
(51) Int. Cl.: A61N 1/37, A61N 1/368

(54) **IMPLANTABLE SYSTEM FOR STIMULATING A HUMAN HEART OR AN ANIMAL HEART**
IMPLANTIERBARES SYSTEM ZUR STIMULATION EINES MENSCHLICHEN ODER TIERISCHEN HERZENS
SYSTÈME IMPLANTABLE POUR STIMULER UN COEUR HUMAIN OU UN COEUR ANIMAL

(30) Priority: 18.11.2019 EP 19209653
(43) Date of publication of application: 28.09.2022
(73) Proprietor: BIOTRONIK SE & Co. KG, 12359 Berlin (DE)
(72) Inventor: DÖRR, Thomas, 12437 Berlin (DE)
(74) Representative: Biotronik Corporate Services SE
(86) International application number: PCT/EP2020/076726
(87) International publication number: WO 2021/099014

(56) References cited:
- WO-A1-2019/089528
- US-A- 5 735 882
- US-A1- 2011 264 158

## Description

The present invention relates to an implantable system for stimulating a human heart or an animal heart according to claim 1, to a method for controlling the operation of such an implantable system as defined in claim 11, and to a computer program product as defined in claim 12.

Implantable systems for stimulating a human heart or an animal heart, such as cardiac pacemakers, have been known for a long time. These can carry out a variety of functions. Various stimulation programs may be carried out by a corresponding cardiac pacemaker in the process so as to return the treated heart to a normal state. Cardiac pacemakers for stimulating the His bundle are also known.

The His bundle is a bundle of specific heart muscle cells that forms part of the cardiac conduction system. The His bundle is located distal from the atrioventricular node, toward the cardiac apex.

Conventional cardiac pacemakers and implantable defibrillators are frequently used for His bundle pacing. A stimulation output of such a conventional device is connected to a His bundle electrode in the process. The conventional devices are then typically adapted to the needs of His bundle pacing within the scope of the available parameter value ranges, with certain concessions.

Conventional devices for stimulating the human or animal heart generally have a safety mode (also referred to as back-up mode). When a fault is detected on the part of the device, the safety mode is activated by using typically established device settings for the continued operation of the device. In general, however, the traditionally utilized device settings do not allow adequate His bundle pacing. For example, it is possible for a His bundle electrode to be connected to the right atrial terminal of a device for stimulating the heart, but for a VVI mode with right ventricular pacing to be stored for this device as the safety mode. In the VVI mode, only right ventricular signals are sensed. In addition, only right ventricular pacing is carried out. Moreover, a stimulation by the cardiac pacemaker is inhibited in the VVI mode when intrinsic activity of the heart is detected. The right atrial terminal, however, is no longer addressed at all in the VVI mode. As a result, His bundle pacing can no longer take place, whereby the patient wearing such a device is then undersupplied.

US 6,996,437 B2 describes various safety modes of a cardiac pacemaker designed for ventricular pacing of a heart. US 5,735,882 shows a cardiac stimulator having a backup mode.

US 8,688,234 B2 describes a cardiac pacemaker suitable for pacing the His bundle of a patient. Different pacing modes are provided in the process. Systems for pacing of a His bundle are also known from WO 2019/089528 A1 and US2011/0264158 A1.

It is the objective of the present invention to provide a device for stimulating a human heart or an animal heart that is able to carry out reliable His bundle pacing even in the event of a fault.

This objective is attained by an implantable system for stimulating a human heart or an animal heart having the features of claim 1.

Such a system comprises a processor, a memory unit (memory device) and a stimulation unit (stimulation device) for stimulating a His bundle or another cardiac region of a human heart or an animal heart. Moreover, such a system comprises a sensing unit (sensing device) for sensing an electrical signal of this heart. Furthermore, a diagnostic unit (diagnostic device) for checking functional parameters of the implantable system is provided.

The memory unit includes a computer-readable program, which prompts the processor to carry out the steps described hereafter when the program is being executed on the processor.

It is thus detected, on the one hand, by way of the diagnostic unit whether at least one malfunction state of the implantable system is present.

On the other hand, it is checked whether an electrode provided for stimulating a His bundle of a human heart or an animal heart is connected to the stimulation unit. In other words, it is checked whether the implantable system is provided for stimulating the His bundle of a patient.

If a malfunction state was detected, the operating state of the implantable system is switched into a safety mode (also referred to as backup mode) according to the executed program. This safety mode can be selected at least from a first and a second safety mode.

The first safety mode is then selected when it was ascertained during the checking as to whether an electrode provided for stimulating the His bundle of the heart that no such electrode is connected. The first safety mode ensures a first safety operation of the implantable system, in which the implantable system does not meet any specific requirements for His bundle pacing. When no electrode that is provided for stimulating the His bundle is connected to the stimulation unit of the implantable system, no safety operation that is specifically tailored to His bundle pacing is required. Rather, for example, it is possible to use a safety mode known from the related art, such as a VVI mode, as the first safety mode.

If, in contrast, it was ascertained during the checking as to whether an electrode suitable for stimulating the His bundle is connected to the stimulation unit that this is the case, the second safety mode is selected. The second safety mode ensures a second safety operation of the implantable system. In this second safety operation, the implantable system is able to meet specific requirements for His bundle pacing. For example, in the second safety operation resulting from the second safety mode, a stimulation pulse that has an amplitude in a range of 7.5 V to 30 V, in particular of 12 V to 30 V, in particular of 15 V to 25 V, and in particular of 20 V to 22 V, and a pulse width in a range of 1 ms to 15 ms, in particular of 2 ms to 12 ms, in particular of 3 ms to 10 ms, in particular of 4 ms to 9 ms, and in particular of 5 ms to 8 ms can be implemented, conducted or provided by the terminal of the stimulation unit to which the electrode provided for stimulating the His bundle is connected.

The presently claimed implantable system is thus designed so as to be able to select and apply different safety modes, as a function of whether or not an electrode suitable for His bundle pacing is connected to the stimulation unit of the system. In this way, in the case where no electrode suitable for His bundle pacing is connected, a safety mode (namely the first safety mode) can be selected, as before, which corresponds to a conventional safety mode and ensures a simple basic function of the implantable system. On the other hand, a different safety mode (namely the second safety mode), which, even though it likewise satisfies the basic functions of the conventional safety mode, moreover allows His bundle pacing as well, can be selected when an electrode suitable for His bundle stimulation is connected to the stimulation unit of the implantable system. This avoids an undersupply situation of a patient occurring with cardiac pacemakers known from the prior art. The second safety mode thus makes it possible to deliver a life-sustaining stimulation therapy of a His bundle stimulator, even if the detection of a malfunction causes a general or other operating state to be switched into a safety mode or safety operation of the implantable system.

In one variant, the implantable system is configured as a cardiac pacemaker. In another variant, this is configured as an implantable cardioverter-defibrillator (ICD). In another variant, the system is configured as a device for carrying out cardiac resynchronization therapy (CRT). In another variant, the implantable system is configured as a device for cardiac resynchronization therapy with ICD function (CRT-D device). In another variant, the system is configured as an implantable leadless pacemaker or pacer (iLP).

In one variant, the information that is required for switching the operating state of the system into the safety mode is stored in a non-volatile memory, a redundant memory or a recoverable memory of the implantable system. This memory may be the memory unit of the implantable system, a memory area of the memory unit of the implantable system or a memory separate from the memory unit. A particularly suitable variant involves storing the pieces of information required for switching in a non-volatile memory. Particularly suitable non-volatile memories are memories of the ROM, EPROM, EEPROM, and FLASH type. It is then possible to ensure particularly easily that the pieces of information required for the switch into the safety mode cannot be inadvertently deleted, but are ready for retrieval at all times. This increases the reliability of the device.

In one variant, the implantable system is able to automatically detect whether an electrode provided for His bundle pacing is connected to the stimulation unit of the system. This can be achieved, for example, by reading out a code of a connector plug of the electrode provided for His bundle pacing.

In another variant, the checking as to whether an electrode that is provided for stimulating the His bundle of a human heart or an animal heart is connected to the stimulation unit is carried out using information that is stored in the memory unit or in another memory of the implantable system. For example, such information can be stored in the memory unit within the setting of the implantation of the implantable system. It is not necessary then for a connector plug of an electrode suitable for His bundle pacing to have a particular configuration. Rather, a surgeon is then able to store information in the implantable system that this system is equipped with such a His bundle stimulation electrode, when implanting an electrode provided for His bundle pacing together with the implantable system. All that is required in this variant is to query the memory of the implantable system as to whether corresponding information has been stored. It is then assumed that such an electrode suitable for His bundle pacing is in fact connected, so that thereafter, in the event of a malfunction, the operating state of the implantable system is switched into the second safety mode.

In one variant, the diagnostic unit is designed so as to be able to detect, based on certain events, whether or not a malfunction state of the implantable system is present. These events in which a malfunction state is detected include, in this variant, a memory error (such as a so-called single bit upset), a software runtime error, a software status error, an detected cyberattack (that is, the attempt of to remotely access the implantable system without authorization), a parameter error, an activation of a reset state of the implantable system (wherein, in general, different reset states of the implantable system are conceivable and settable), a battery drainage (that is, reaching or dropping below a pre-definable capacity limit of a battery of the implantable system at which a flawless operation of the system is no longer ensured), and outside electromagnetic interference of the implantable system.

Such outside electromagnetic interference can be a strong permanent magnetic field, for example, as it is emitted by magnetic resonance imaging (MRI) machines. If a patient knows, for example, that an MRI examination is to be carried out within a certain time frame, a physician can store corresponding information in the implantable system. Since strong magnetic fields can impair the function of the implantable system, the implantable system is sensitized, during a pre-definable time period, with increased monitoring accuracy to the detection as to whether the system is exposed to a permanent magnetic field. If this is the case, one of the safety modes can be selected directly and activated, so as to prevent a malfunction of the implantable system. The detection of such an MRI environment can thus be identified as a malfunction state and result in the activation of one of the safety modes. After the MRI examination has been completed, the implantable system can then be manually transferred back into the customary operating state.

In one variant, the second safety operation is characterized by at least one of the configurations described hereafter.

For example, a specific His bundle pacing setting of an electrode terminal of the stimulation unit, to which an electrode provided for His bundle pacing is connected, can be implemented during the second safety operation as a configuration. The electrode terminal to which the His bundle electrode is connected can, for example, be selected, as described above, using user-dependent information.

As an alternative or in addition, stimulation energy for a stimulation, which can be delivered by a His bundle electrode for stimulating a His bundle, can be set specifically so as to be suitable for stimulating the His bundle of the particular heart. Suitable stimulation energies (characterized by voltage and pulse widths) are described above.

As an alternative or in addition, a detection sensitivity (sensing sensitivity) of the sensing unit for sensing His bundle-specific signals can be set. It can then be ensured that His bundle-specific signals can in fact be correctly sensed in the safety operation.

As an alternative or in addition, a stimulation operating mode of the implantable system can be set to an operating mode specific to His bundle pacing. This can take place, for example, by specifying certain parameter ranges, which relate to different functions of the implantable system (in particular stimulation, sensing and/or storage of the sensed or executed events).

As an alternative or in addition, a stimulation frequency (stimulation rate) of a stimulation can be set, which can be delivered by an electrode suitable for His bundle pacing.

As an alternative or in addition, it is also possible to implement a specific His bundle pacing setting of an additional electrode terminal of the stimulation unit to which an electrode provided for stimulating the His bundle of a human heart or an animal heart is additionally connected (so-called His bundle back-up stimulation terminal).

In one variant, the program prompts the processor to additionally carry out the step described hereafter. In this additional step, an operating state of the implantable system is switched into a protective mode when the protective mode was activated by an external signal. In contrast to a safety mode, which is activated by a device-internal diagnosis of the function of the implantable system, the protective mode is thus a mode that can be activated by an external signal. The protective mode could, for example, be activated by a programming device emergency function. This can be carried out by a physician, for example, when the physician establishes during an examination that the implantable system does not function in the intended manner.

According to one embodiment, the protective mode represents a standardized, uniform safe operating mode for the stimulator, which can be used for all patients wearing an implant designed for His pacing. In contrast, the safety mode can, for example, depend on an individual device setting and be configured in a patient-specific manner.

The protective mode is then selected at least from a first and a second protective mode.

Similarly to the first safety mode, the first protective mode is then selected when the prior check has shown that no electrode provided for stimulating the His bundle of a human heart or an animal heart is connected to the stimulation unit. The first protective mode causes the implantable system to be transferred into a first protective operation, in which the implantable system does not meet any specific requirements for His bundle pacing. The reason is that when no His bundle electrode is connected to the stimulation unit of the system, it is not necessary to consider specific requirements for His bundle pacing.

In contrast, the second protective mode is selected when the prior checking as to whether an electrode suitable for His bundle pacing is connected to the stimulation unit has shown that such an electrode is present. The second protective mode activates a second protective operation of the implantable system, in which the implantable system is able to meet the specific requirements for His bundle pacing. The second protective mode is comparable to the second safety mode. In particular, all variants that are described in the present application with respect to the second safety mode can thus also be directly applied to the second protective mode.

In one variant, the second protective operation is thus characterized by at least one of the following configurations: a His bundle pacing setting of an electrode terminal of the stimulation unit to which an electrode provided for stimulating a His bundle of a human heart or an animal heart is connected; a stimulation energy of a stimulation to be delivered by an electrode provided for stimulating a His bundle of a human heart or an animal heart; a sensing sensitivity of the sensing unit for sensing His bundle-specific signals; a stimulation operating mode of the implantable system; a stimulation frequency of a stimulation to be delivered by an electrode provided for stimulating a His bundle of a human heart or an animal heart; and a His bundle pacing setting of a second electrode terminal of the stimulation unit to which a second electrode for stimulating a human heart or an animal heart is connected. The second electrode can, for example, be a right or left ventricular stimulation electrode, and the second electrode terminal can accordingly be the terminal for a right or left ventricular stimulation electrode.

In one variant, it is possible for the configuration of the second safety operation (and/or of the second protective operation) to be establishable or individually adaptable as a function of settable programming parameters. For this purpose, it is possible for a user to adapt the corresponding programming parameters to the needs of a patient in whom the implantable system was implanted. It is then possible to address patient-specific circumstances, thereby enabling an individual safety operation or protective operation of the system.

In one variant, pieces of information are stored in the implantable system, based on which the configuration of the second safety operation (and/or the second protective operation) is automatically determined. This means that, in this variant, no setting options of parameters with respect to the configuration of the second safety operation are provided. This variant eliminates the risk of inadvertently incorrectly setting certain parameters since the second safety operation (and/or the second protective operation) is always carried out using preset parameters.

In one variant, the implantable system comprises a data remote transmission unit. **In** this variant, the program can prompt the processor to transmit an activation of the safety mode to a remote monitoring system by means of the data remote transmission unit. It is then possible to detect in the remote monitoring system that a malfunction state was sensed in the implantable system, which resulted in the safety mode being activated. The patient in whom the implantable system was implanted and/or the physician can then be notified that it is necessary to check the function of the implantable system.

In one variant, it is displayed on a programming device, which can be used to program the implantable system, that the safety mode was activated. It can also be displayed in the process which safety mode was activated. In this way, it is possible to check particularly easily whether the settings that are stored for the particular implantable system with respect to the presence of an electrode suitable for His bundle pacing are in fact correct. In addition, it is also possible to check particularly easily whether an activation or inactivation of the safety mode was successful.

One aspect of the present invention relates to a method for controlling the operation of an implantable system for stimulating a human heart or an animal heart as defined in claim 11.

This method is characterized by the steps described hereafter.

In one method step, it is detected, by means of a diagnostic unit for checking functional parameters of the implantable system, whether at least one malfunction state of the implantable system is present.

In a further method step, it is checked whether an electrode, which is provided for stimulating a His bundle of a human heart or an animal heart, is connected to a stimulation unit of the implantable system. This stimulation unit is designed to stimulate a His bundle or another cardiac region of a human heart or an animal heart.

In a further method step, an operating state of the implantable system is switched into a safety mode when a malfunction state was detected. The safety mode can be selected at least from a first and a second safety mode in the process.

The first safety mode is then selected when the previously completed check has shown that no electrode provided for stimulating a His bundle of a human heart or an animal heart is connected to the stimulation unit. The first safety mode results in the activation of a first safety operation of the implantable system, in which the implantable system does not meet any specific requirements for His bundle pacing. The reason is that this is generally not required when no electrode at all that is provided for His bundle pacing is connected to the stimulation unit of the system.

In contrast, the second safety mode is selected when the previously completed check has shown that an electrode that is suitable for stimulating a His bundle of a human heart or an animal heart is connected to the stimulation unit. The second safety mode causes a second safety operation of the implantable system, in which the implantable system is able to meet specific requirements for His bundle pacing.

One aspect of the present invention relates to a computer program product including computer-readable code, which prompts the processor of the implantable system according to any of claims 1-10 to carry out the steps described hereafter when the code is being executed on the processor.

In a first step, it is detected, by means of a diagnostic unit for checking functional parameters of the implantable system, whether at least one malfunction state of the implantable system is present.

In a further -step, it is checked whether an electrode, which is provided for stimulating a His bundle of a human heart or an animal heart, is connected to a stimulation unit of the implantable system. This stimulation unit is designed to stimulate a His bundle or another cardiac region of a human heart or an animal heart.

In a further step, an operating state of the implantable system is switched into a safety mode when a malfunction state was detected. The safety mode can be selected at least from a first and a second safety mode in the process.

The first safety mode is then selected when the previously completed check has shown that no electrode provided for stimulating a His bundle of a human heart or an animal heart is connected to the stimulation unit. The first safety mode results in the activation of a first safety operation of the implantable system, in which the implantable system does not meet any specific requirements for His bundle pacing. The reason is that this is generally not required when no electrode at all that is provided for His bundle pacing is connected to the stimulation unit of the system.

In contrast, the second safety mode is selected when the previously completed check has shown that an electrode that is suitable for stimulating a His bundle of a human heart or an animal heart is connected to the stimulation unit. The second safety mode causes a second safety operation of the implantable system, in which the implantable system is able to meet specific requirements for His bundle pacing.

The present invention can be used in an exemplary, non-claimed method for treating a human patient or an animal patient in need of such treatment. This treatment is carried out by means of an implantable system for stimulating a human heart or an animal heart, and in particular by means of an implantable system according to the above description. Such an implantable system comprises a stimulation unit for stimulating a His bundle or another cardiac region of a human heart or an animal heart. This furthermore comprises a sensing unit for sensing an electrical signal of this heart. Finally, such a system is equipped with a diagnostic unit, which is used to check functional parameters of the implantable system. The medical method comprises the steps described hereafter:
In one method step, the diagnostic unit detects whether at least one malfunction state of the implantable system is present.

In a further method step, it is checked whether an electrode, which is provided for stimulating a His bundle of a human heart or an animal heart, is connected to the stimulation unit.

In a further method step, an operating state of the implantable system is switched into a safety mode when a malfunction state was detected. The safety mode can be selected at least from a first and a second safety mode in the process.

The first safety mode is then selected when the previously completed check has shown that no electrode provided for stimulating a His bundle of a human heart or an animal heart is connected to the stimulation unit. The first safety mode results in the activation of a first safety operation of the implantable system, in which the implantable system does not meet any specific requirements for His bundle pacing. The reason is that this is generally not required when no electrode at all that is provided for His bundle pacing is connected to the stimulation unit of the system.

In contrast, the second safety mode is selected when the previously completed check has shown that an electrode that is suitable for stimulating a His bundle of a human heart or an animal heart is connected to the stimulation unit. The second safety mode causes a second safety operation of the implantable system, in which the implantable system is able to meet specific requirements for His bundle pacing.

If a corresponding medical need exists, the human or animal heart of the patient in whom the implantable system was implanted is then stimulated using the implantable system. The stimulation is carried out as part of the safety operation of the implantable system, which is specified by the previously selected safety mode.

The present invention is defined by the appended claims.

Details of aspects of the present invention are to be described in more detail based on exemplary embodiments and figures. In the drawings:
- FIG. 1: shows a schematic illustration of different electrode configurations for connecting an electrode suitable for His bundle pacing to an implantable system for stimulating the human or animal heart; and
- FIG. 2: shows a schematic flow chart of a method for controlling the operation of an implantable system for stimulating the human heart or an animal heart.

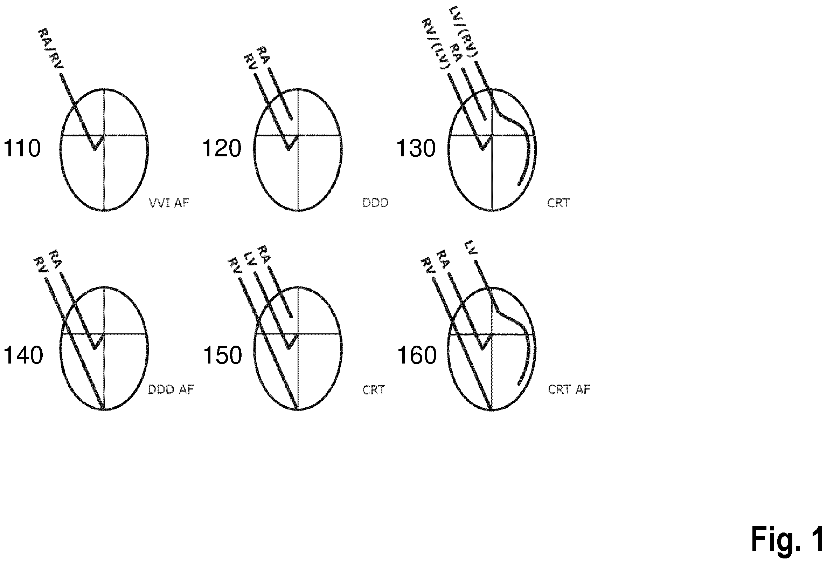

FIG. 1 shows a schematic illustration of different electrode configurations that are possible for connecting an electrode suitable for stimulating the His bundle of a human heart or an animal heart to a device for stimulating this heart.

A single-chamber stimulation is carried out in a first electrode configuration 110. For this purpose, the electrode suitable for His bundle pacing is connected to an electrode terminal for an electrode for stimulating the right atrium RA or for stimulating the right ventricle RV. Such a pacemaker can also be referred to as a single-chamber pacemaker or a demand pacemaker. At least in the safety mode, it is typically operated in a mode in which a stimulation takes place in the right ventricle, and sensing takes place in the right ventricle, and an inhibiting operating mode is carried out (VVI). This means that the pacemaker only applies pacing pulses when no intrinsic activity of the heart can be detected. In this mode, a right ventricular or biventricular stimulation is possible using the His bundle electrode.

When the electrode provided for His bundle pacing is connected to the RA terminal of such a pacemaker, and such a pacemaker enters a VVI safety mode, adequate His bundle pacing is not possible. The presently described invention is thus suitable, in particular, for improving cardiac pacemakers having the first electrode configuration 110. However, further electrode configurations described hereafter also typically result in an undersupply situation of the patient as a result of the selection of conventional safety modes. The presently claimed invention also offers a corrective action in this regard by ensuring a safety mode configured specifically for His bundle pacing and allowing this safety mode to be activated when a His bundle electrode is connected.

In a second electrode configuration 120, the electrode used for stimulating the His bundle is connected to the terminal of the right ventricle RV. In addition, an arterial electrode is also connected to the terminal for the right atrium RA. Such a pacemaker can be operated in the DDD mode (dual pacing (that is, atrial and ventricular), dual sensing (that is, atrial and ventricular) with dual operating mode (that is, triggering or inhibition). In this mode as well, a right ventricular or biventricular stimulation is possible using the His bundle electrode. As a result, sequential pacing of the atrium and of the His bundle is possible.

In a third electrode configuration 130, the electrode suitable for stimulating the His bundle is connected to the terminal for the right ventricle RV or the left ventricle LV of the cardiac pacemaker. In contrast, an electrode provided for stimulating the left ventricle is connected to the, still free, terminal for stimulating the left ventricle LV or the right ventricle RV. Moreover, an atrial electrode is connected to the terminal for the right atrium RA. Such a stimulation device is, in particular, a device for cardiac resynchronization therapy (CRT). It allows biventricular stimulation as well as cardiac resynchronization with optional His bundle pacing.

In a fourth electrode configuration 140, the electrode used for stimulating the His bundle is connected to the terminal for the right atrium RA of a pacemaker. Moreover, an electrode for stimulating the right ventricle is connected to the corresponding terminal for the right ventricle RV. Such a pacemaker can implement an RV safety mode and is, in particular, suited for operation in the DDD mode.

In a fifth electrode configuration 150, the electrode provided for His bundle pacing is connected to the terminal for the left ventricle LV. Moreover, an atrial electrode is connected to the terminal for the right atrium RA, and a ventricular electrode is connected to the terminal for the right ventricle RV. Such a device is also in particular a CRT device, which allows right ventricular or biventricular stimulation, and moreover enables optional His bundle pacing when carrying out cardiac resynchronization therapy.

In a sixth electrode configuration 160, the electrode provided for His bundle pacing is, again, connected to the terminal for the right atrium RA. Moreover, two ventricular electrodes are provided, of which the electrode arranged in the right ventricle is connected to the terminal for the right ventricle RV, and the left ventricular electrode is connected to the terminal for the left ventricle LV. Once again, such an electrode configuration is particularly suited for CRT devices, which are able to carry out a biventricular stimulation and cardiac resynchronization with optional His bundle pacing.

An implementation of the presently claimed invention is useful and contemplated in the various stimulation devices whose electrode configurations are shown in FIG. 1. The reason is that the problem of the patient possibly being undersupplied when conventional safety modes are selected arises in all of these electrode configurations. The option of selecting a safety mode that is tailored to the specific requirements of His bundle pacing makes it possible to avoid such an undersupply situation when an electrode suitable for His bundle pacing is connected to the corresponding stimulation device.

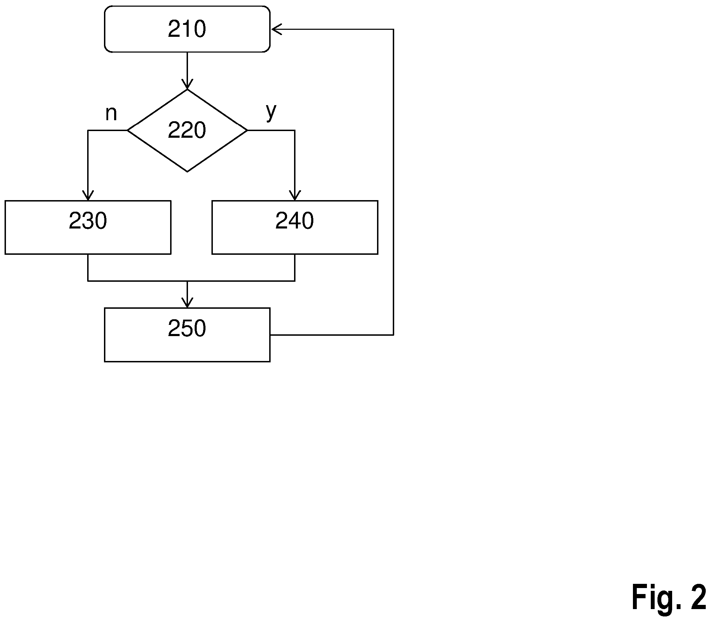

FIG. 2 shows a schematic flow chart of a method for controlling the operation of an implantable system for stimulating the animal or human heart, which can implement the second safety mode according to the invention (hereafter referred to as stimulation device).

This stimulation device is initially active in a regular operating mode 210. When an error state is detected as a result a self-test carried out cyclically by a corresponding diagnostic unit, a check 220 is carried out as to whether an electrode suitable for stimulating the His bundle is connected to the stimulation device. This can be achieved, for example, by querying internal information, which is stored in the stimulation device when an electrode suitable for His bundle pacing is connected.

If the check 220 shows that no electrode suitable for His bundle pacing is connected to the stimulation device, a first safety mode 230 is selected, by way of which thereafter a first safety operation of the stimulation device is carried out.

If, in contrast, the check 220 shows that an electrode provided for His bundle pacing is connected to the stimulation device, a second safety mode 240 is selected, which transfers the stimulation device into a second safety operation. This second safety operation is specifically tailored to the needs of His bundle pacing, whereby adequate His bundle pacing is also possible in this safety mode.

After a restoration procedure 250, which, in particular, removes the error state that results in the stimulation device being transferred into one of the safety modes in the first place, the stimulation device is transferred back into the regular operating mode 210 thereof.

## Claims

1. An implantable system for stimulating a human heart or an animal heart, comprising a processor, a memory unit, a stimulation unit for stimulating a His bundle or another cardiac region of a human heart or an animal heart, a sensing unit for sensing an electrical signal of this heart, and a diagnostic unit for checking functional parameters of the implantable system,
wherein the memory unit includes a computer-readable program, which prompts the processor to carry out the following steps when the program is being executed on the processor:
a) detecting, by means of the diagnostic unit, whether at least one malfunction state of the implantable system is present;
**characterised by** the following steps:
b) checking (220) whether an electrode provided for stimulating a His bundle of a human heart or an animal heart is connected to the stimulation unit; and
c) switching an operating state of the implantable system into a safety mode when a malfunction state was detected, the safety mode being selected at least from a first safety mode (230) and a second safety mode (240), wherein
i) the first safety mode (230) is selected when the checking (220) carried out in step b) has shown that no electrode provided for stimulating a His bundle of a human heart or an animal heart is connected to the stimulation unit, the first safety mode (230) resulting in a first safety operation of the implantable system, in which the implantable system does not meet any specific requirements for His bundle pacing; and
ii) the second safety mode (240) is selected when the checking (220) carried out in step b) has shown that an electrode provided for stimulating a His bundle of a human heart or an animal heart is connected to the stimulation unit, the second safety mode (240) resulting in a second safety operation of the implantable system in which the implantable system is able to meet specific requirements for His bundle pacing.

2. The implantable system according to claim 1, **characterized in that** pieces of information that are required for switching the system into the safety mode are stored in a non-volatile memory, a redundant memory or a recoverable memory of the implantable system.

3. The implantable system according to claim 1 or 2, **characterized in that** the checking (220) as to whether an electrode provided for stimulating a His bundle of a human heart or an animal heart is connected to the stimulation unit is carried out based on information stored in the memory unit.

4. The implantable system according to any one of the preceding claims, **characterized in that** the diagnostic unit is configured to detect the malfunction state based on at least one of the following events: a memory error, a software runtime error, a software status error, a detected cyberattack, a parameter error, an activation of a reset state of the implantable system, a battery drainage, and outside electromagnetic interference of the implantable system.

5. An implantable system according to any one of the preceding claims, **characterized in that** the second safety operation is **characterized by** at least one of the following configurations: a His bundle pacing setting of an electrode terminal of the stimulation unit to which an electrode provided for stimulating a His bundle of a human heart or an animal heart is connected; a stimulation energy of a stimulation to be delivered by an electrode provided for stimulating a His bundle of a human heart or an animal heart; a sensing sensitivity of the sensing unit for sensing His bundle-specific signals; a stimulation operating mode of the implantable system; a stimulation frequency of a stimulation to be delivered by an electrode provided for stimulating a His bundle of a human heart or an animal heart; and a His bundle pacing setting of a second electrode terminal of the stimulation unit to which a second electrode for stimulating a human heart or an animal heart is connected.

6. An implantable system according to any one of the preceding claims, **characterized in that** the program prompts the processor to additionally carry out the following step:
a) switching an operating state of the implantable system into a protective mode when the protective mode was activated by an external signal, the protective mode being selected at least from a first and a second protective mode, wherein
i) the first protective mode is selected when the checking (220) carried out in step b) has shown that no electrode provided for stimulating a His bundle of a human heart or an animal heart is connected to the stimulation unit, the first protective mode resulting in a first protective operation of the implantable system in which the implantable system does not meet any specific requirements for His bundle pacing; and
ii) the second protective mode is selected when the checking carried out in step b) has shown that an electrode provided for stimulating a His bundle of a human heart or an animal heart is connected to the stimulation unit, the second protective mode resulting in a second protective operation of the implantable system in which the implantable system is able to meet specific requirements for His bundle pacing.

7. An implantable system according to any one of the preceding claims, **characterized in that** the second safety operation is **characterized by** at least one of the following configurations: a His bundle pacing setting of an electrode terminal of the stimulation unit to which an electrode provided for stimulating a His bundle of a human heart or an animal heart is connected; a stimulation energy of a stimulation to be delivered by an electrode provided for stimulating a His bundle of a human heart or an animal heart; a sensing sensitivity of the sensing unit for sensing His bundle-specific signals; a stimulation operating mode of the implantable system; a stimulation frequency of a stimulation to be delivered by an electrode provided for stimulating a His bundle of a human heart or an animal heart; and a His bundle pacing setting of an additional electrode terminal of the stimulation unit to which an electrode provided for stimulating a His bundle of a human heart or an animal heart is connected.

8. The implantable system according to any one of the preceding claims, **characterized in that** the program prompts the processor to establish or individually adapt the configuration of the second safety operation as a function of settable programming parameters.

9. The implantable system according to any one of claims 1 to 7, **characterized in that** the program prompts the processor to automatically determine the configuration of the second safety operation as a function of pieces of information stored in the implantable system.

10. The implantable system according to any one of the preceding claims, **characterized in that** the implantable system comprises a data remote transmission unit, the program prompting the processor to transmit an activation of the safety mode to a remote monitoring system by means of the data remote transmission unit.

11. A method for controlling the operation of an implantable system for stimulating a human heart or an animal heart according to any one of the preceding claims, wherein the method comprises the following steps:
a) detecting, by means of the diagnostic unit for checking functional parameters of the implantable system, whether at least one malfunction state of the implantable system is present;
b) checking (220) whether an electrode provided for stimulating a His bundle of a human heart or an animal heart is connected to the stimulation unit for stimulating a His bundle or a similar cardiac region of a human heart or an animal heart; and
c) switching an operating state of the implantable system into a safety mode when a malfunction state was detected, the safety mode being selected at least from a first safety mode (230) and a second safety mode (240), wherein
i) the first safety mode (230) is selected when the checking (220) carried out in step b) has shown that no electrode provided for stimulating a His bundle of a human heart or an animal heart is connected to the stimulation unit, the first safety mode (230) resulting in a first safety operation of the implantable system in which the implantable system does not meet any specific requirements for His bundle pacing; and
ii) the second safety mode (240) is selected when the checking (220) carried out in step b) has shown that an electrode provided for stimulating a His bundle of a human heart or an animal heart is connected to the stimulation unit, the second safety mode (240) resulting in a second safety operation of the implantable system in which the implantable system is able to meet specific requirements for His bundle pacing.

12. A computer program product including computer-readable code, which prompts the processor of the implantable system according to any one of claims 1-10 to carry out the following steps when the code is being executed on the processor:
a) detecting, by means of the diagnostic unit for checking functional parameters of the implantable system for stimulating a human heart or an animal heart, whether at least one malfunction state of the implantable system is present;
b) checking (220) whether an electrode provided for stimulating a His bundle of a human heart or an animal heart is connected to the stimulation unit for stimulating a His bundle or a similar cardiac region of a human heart or an animal heart; and
c) switching an operating state of the implantable system into a safety mode when a malfunction state was detected, the safety mode being selected at least from a first safety mode (230) and a second safety mode (240), wherein
i) the first safety mode (230) is selected when the checking (220) carried out in step b) has shown that no electrode provided for stimulating a His bundle of a human heart or an animal heart is connected to the stimulation unit, the first safety mode (230) resulting in a first safety operation of the implantable system in which the implantable system does not meet any specific requirements for His bundle pacing; and
ii) the second safety mode (240) is selected when the checking (220) carried out in step b) has shown that an electrode provided for stimulating a His bundle of a human heart or an animal heart is connected to the stimulation unit, the second safety mode (240) resulting in a second safety operation of the implantable system in which the implantable system is able to meet specific requirements for His bundle pacing.

## Patentansprüche

1. Implantierbares System zur Stimulation eines menschlichen Herzens oder eines tierischen Herzens, umfassend einen Prozessor, eine Speichereinheit, eine Stimulationseinheit zum Stimulieren eines His-Bündels oder eines anderen Herzbereichs eines menschlichen Herzens oder eines tierischen Herzens, eine Erfassungseinheit zum Erfassen eines elektrischen Signals von diesem Herzen und eine Diagnoseeinheit zum Prüfen von funktionalen Parametern des implantierbaren Systems,
wobei die Speichereinheit ein computerlesbares Programm enthält, das den Prozessor dazu veranlasst, die folgenden Schritte auszuführen, wenn das Programm auf dem Prozessor ausgeführt wird:
a) Detektieren, mittels der Diagnoseeinheit, ob mindestens ein Fehlfunktionszustand des implantierbaren Systems vorliegt;
**gekennzeichnet durch** die folgenden Schritte:
b) Prüfen (220), ob eine Elektrode, die zur Stimulation eines His-Bündels eines menschlichen Herzens oder eines tierischen Herzens vorgesehen ist, mit der Stimulationseinheit verbunden ist; und
c) Schalten eines Betriebszustands des implantierbaren Systems in einen Sicherheitsmodus wenn ein Fehlfunktionszustand detektiert wurde, wobei der Sicherheitsmodus zumindest aus einem ersten Sicherheitsmodus (230) und einem zweiten Sicherheitsmodus (240) ausgewählt wird, wobei
i) der erste Sicherheitsmodus (230) ausgewählt wird, wenn das in Schritt b) ausgeführte Prüfen (220) gezeigt hat, dass keine Elektrode, die zur Stimulation eines His-Bündels eines menschlichen Herzens oder eines tierischen Herzens vorgesehen ist, mit der Stimulationseinheit verbunden ist, wobei der erste Sicherheitsmodus (230) zu einem ersten Sicherheitsbetrieb des implantierbaren Systems führt, bei dem das implantierbare System keine bestimmten Anforderungen für die Stimulation eines His-Bündels erfüllt; und
ii) der zweite Sicherheitsmodus (240) ausgewählt wird, wenn das in Schritt b) ausgeführte Prüfen (220) gezeigt hat, dass eine Elektrode, die zur Stimulation eines His-Bündels eines menschlichen Herzens oder eines tierischen Herzens vorgesehen ist, mit der Stimulationseinheit verbunden ist, wobei der zweite Sicherheitsmodus (240) zu einem zweiten Sicherheitsbetrieb des implantierbaren Systems führt, bei dem das implantierbare System bestimmte Anforderungen für die Stimulation eines His-Bündels erfüllen kann.

2. Implantierbares System nach Anspruch 1, **dadurch gekennzeichnet, dass** Teile der zum Schalten des Systems in den Sicherheitsmodus erforderlichen Informationen in einem nichtflüchtigen Speicher, einem redundanten Speicher oder einem wiederherstellbaren Speicher des implantierbaren Systems gespeichert werden.

3. Implantierbares System nach Anspruch 1 oder 2, **dadurch gekennzeichnet, dass** das Prüfen (220), ob eine Elektrode, die zur Stimulation eines His-Bündels eines menschlichen Herzens oder eines tierischen Herzens vorgesehen ist, mit der Stimulationseinheit verbunden ist, basierend auf in der Speichereinheit gespeicherten Informationen durchgeführt wird.

4. Implantierbares System nach einem der vorhergehenden Ansprüche, **dadurch gekennzeichnet, dass** die Diagnoseeinheit dazu konfiguriert ist, den Fehlfunktionszustand basierend auf mindestens einem der folgenden Ereignisse zu detektieren: einem Speicherfehler, einem Softwarelaufzeitfehler, einem Softwarezustandsfehler, einem detektierten Cyberangriff, einem Parameterfehler, einer Aktivierung eines Rücksetzzustands des implantierbaren Systems, einer Batterieentladung und einer von außen kommenden elektromagnetischen Störbeeinflussung des implantierbaren Systems.

5. Implantierbares System nach einem der vorhergehenden Ansprüche, **dadurch gekennzeichnet, dass** der zweite Sicherheitsbetrieb durch mindestens eine der folgenden Konfigurationen gekennzeichnet ist: eine Einstellung der Stimulation eines His-Bündels für ein Elektrodenpol der Stimulationseinheit, mit der eine Elektrode, die zur Stimulation eines His-Bündels eines menschlichen Herzens oder eines tierischen Herzens vorgesehen ist, verbunden ist; eine Stimulationsenergie einer Stimulation, die von einer Elektrode übertragen werden soll, die zur Stimulation eines His-Bündels eines menschlichen Herzens oder eines tierischen Herzens vorgesehen ist; eine Erfassungssensitivität der Erfassungseinheit zum Erfassen von His-Bündel-spezifischen Signalen; ein Stimulationsbetriebsmodus des implantierbaren Systems; eine Stimulationsfrequenz einer Stimulation, die von einer Elektrode übertragen werden soll, die zur Stimulation eines His-Bündels eines menschlichen Herzens oder eines tierischen Herzens vorgesehen ist; und eine Einstellung der Stimulation eines His-Bündels für ein zweites Elektrodenpol der Stimulationseinheit, mit der eine zweite Elektrode zur Stimulation eines menschlichen Herzens oder eines tierischen Herzens verbunden ist.

6. Implantierbares System nach einem der vorhergehenden Ansprüche, **dadurch gekennzeichnet, dass** das Programm den Prozessor dazu veranlasst, zusätzlich den folgenden Schritt auszuführen:
a) Schalten eines Betriebszustands des implantierbaren Systems in einen Schutzmodus, wenn der Schutzmodus durch ein externes Signal aktiviert wurde, wobei der Schutzmodus zumindest aus einem ersten und einem zweiten Schutzmodus ausgewählt wird, wobei
i) der erste Schutzmodus ausgewählt wird, wenn das in Schritt b) durchgeführte Prüfen (220) gezeigt hat, dass keine Elektrode, die zur Stimulation eines His-Bündels eines menschlichen Herzens oder eines tierischen Herzens vorgesehen ist, mit der Stimulationseinheit verbunden ist, wobei der erste Schutzmodus zu einem ersten Schutzbetrieb des implantierbaren Systems führt, bei dem das implantierbare System keine bestimmten Anforderungen für die Stimulation eines His-Bündels erfüllt; und
ii) der zweite Schutzmodus ausgewählt wird, wenn das in Schritt b) ausgeführte Prüfen gezeigt hat, dass eine Elektrode, die zur Stimulation eines His-Bündels eines menschlichen Herzens oder eines tierischen Herzens vorgesehen ist, mit der Stimulationseinheit verbunden ist, wobei der zweite Schutzmodus zu einem zweiten Schutzbetrieb des implantierbaren Systems führt, bei dem das implantierbare System bestimmte Anforderungen für die Stimulation eines His-Bündels erfüllen kann.

7. Implantierbares System nach einem der vorhergehenden Ansprüche, **dadurch gekennzeichnet, dass** der zweite Sicherheitsbetrieb durch mindestens eine der folgenden Konfigurationen gekennzeichnet ist: eine Einstellung der Stimulation eines His-Bündels für ein Elektrodenpol der Stimulationseinheit, mit der eine Elektrode, die zur Stimulation eines His-Bündels eines menschlichen Herzens oder eines tierischen Herzens vorgesehen ist, verbunden ist; eine Stimulationsenergie einer Stimulation, die von einer Elektrode übertragen werden soll, die zur Stimulation eines His-Bündels eines menschlichen Herzens oder eines tierischen Herzens vorgesehen ist; eine Erfassungssensitivität der Erfassungseinheit zum Erfassen von His-Bündel-spezifischen Signalen; ein Stimulationsbetriebsmodus des implantierbaren Systems; eine Stimulationsfrequenz einer Stimulation, die von einer Elektrode übertragen werden soll, die zur Stimulation eines His-Bündels eines menschlichen Herzens oder eines tierischen Herzens vorgesehen ist; und eine Einstellung der Stimulation eines His-Bündels für ein zusätzliches Elektrodenpol der Stimulationseinheit, mit der eine Elektrode, die zur Stimulation eines His-Bündels eines menschlichen Herzens oder eines tierischen Herzens vorgesehen ist, verbunden ist.

8. Implantierbares System nach einem der vorhergehenden Ansprüche, **dadurch gekennzeichnet, dass** das Programm den Prozessor dazu veranlasst, die Konfiguration des zweiten Sicherheitsbetriebs als eine Funktion von einstellbaren Programmierparametern einzurichten oder diese individuell anzupassen.

9. Implantierbares System nach einem der Ansprüche 1 bis 7, **dadurch gekennzeichnet, dass** das Programm den Prozessor dazu veranlasst, automatisch die Konfiguration des zweiten Sicherheitsbetriebs als eine Funktion von in dem implantierbaren System gespeicherten Teilen von Informationen zu bestimmen.

10. Implantierbares System nach einem der vorhergehenden Ansprüche, **dadurch gekennzeichnet, dass** das implantierbare System eine Datenfernübertragungseinheit umfasst, wobei das Programm den Prozessor dazu veranlasst, die Aktivierung des Sicherheitsmodus mittels der Datenfernübertragungseinheit an ein entferntes Überwachungssystem zu übertragen.

11. Verfahren zum Steuern des Betriebs eines implantierbaren Systems zur Stimulation eines menschlichen Herzens oder eines tierischen Herzens nach einem der vorhergehenden Ansprüche, wobei das Verfahren die folgenden Schritte umfasst:
a) Detektieren, mittels der Diagnoseeinheit zum Prüfen der funktionalen Parameter des implantierbaren Systems, ob mindestens ein Fehlfunktionszustand des implantierbaren Systems vorliegt;
b) Prüfen (220), ob eine Elektrode, die zur Stimulation eines His-Bündels eines menschlichen Herzens oder eines tierischen Herzens vorgesehen ist, mit der Stimulationseinheit zur Stimulation eines His-Bündels oder eines ähnlichen Herzbereichs eines menschlichen Herzens oder eines tierischen Herzens verbunden ist; und
c) Schalten eines Betriebszustands des implantierbaren Systems in einen Sicherheitsmodus, wenn ein Fehlfunktionszustand detektiert wurde, wobei der Sicherheitsmodus zumindest aus einem ersten Sicherheitsmodus (230) und eine zweiten Sicherheitsmodus (240) ausgewählt wird, wobei
i) der erste Sicherheitsmodus (230) ausgewählt wird, wenn das in Schritt b) ausgeführte Prüfen (220) gezeigt hat, dass keine Elektrode, die zur Stimulation eines His-Bündels eines menschlichen Herzens oder eines tierischen Herzens vorgesehen ist, mit der Stimulationseinheit verbunden ist, wobei der erste Sicherheitsmodus (230) zu einem ersten Sicherheitsbetrieb des implantierbaren Systems führt, bei dem das implantierbare System keine bestimmten Anforderungen für die Stimulation eines His-Bündels erfüllt; und
ii) der zweite Sicherheitsmodus (240) ausgewählt wird, wenn das in Schritt b) ausgeführte Prüfen (220) gezeigt hat, dass eine Elektrode, die zur Stimulation eines His-Bündels eines menschlichen Herzens oder eines tierischen Herzens vorgesehen ist, mit der Stimulationseinheit verbunden ist, wobei der zweite Sicherheitsmodus (240) zu einem zweiten Sicherheitsbetrieb des implantierbaren Systems führt, bei dem das implantierbare System bestimmte Anforderungen für die Stimulation eines His-Bündels erfüllen kann.

12. Computerprogrammprodukt, das computerlesbaren Code enthält, der den Prozessor des implantierbaren Systems nach einem der Ansprüche 1-10 dazu veranlasst, die folgenden Schritte auszuführen, wenn der Code auf dem Prozessor ausgeführt wird:
a) Detektieren, mittels der Diagnoseeinheit zum Prüfen der funktionalen Parameter des implantierbaren Systems zur Stimulation eines menschlichen Herzens oder eines tierischen Herzens, ob mindestens ein Fehlfunktionszustand des implantierbaren Systems vorliegt;
b) Prüfen (220), ob eine Elektrode, die zur Stimulation eines His-Bündels eines menschlichen Herzens oder eines tierischen Herzens vorgesehen ist, mit der Stimulationseinheit zur Stimulation eines His-Bündels oder eines ähnlichen Herzbereichs eines menschlichen Herzens oder eines tierischen Herzens verbunden ist; und
c) Schalten eines Betriebszustands des implantierbaren Systems in einen Sicherheitsmodus, wenn ein Fehlfunktionszustand detektiert wurde, wobei der Sicherheitsmodus zumindest aus einem ersten Sicherheitsmodus (230) und eine zweiten Sicherheitsmodus (240) ausgewählt wird, wobei
i) der erste Sicherheitsmodus (230) ausgewählt wird, wenn das in Schritt b) ausgeführte Prüfen (220) gezeigt hat, dass keine Elektrode, die zur Stimulation eines His-Bündels eines menschlichen Herzens oder eines tierischen Herzens vorgesehen ist, mit der Stimulationseinheit verbunden ist, wobei der erste Sicherheitsmodus (230) zu einem ersten Sicherheitsbetrieb des implantierbaren Systems führt, bei dem das implantierbare System keine bestimmten Anforderungen für die Stimulation eines His-Bündels erfüllt; und
ii) der zweite Sicherheitsmodus (240) ausgewählt wird, wenn das in Schritt b) ausgeführte Prüfen (220) gezeigt hat, dass eine Elektrode, die zur Stimulation eines His-Bündels eines menschlichen Herzens oder eines tierischen Herzens vorgesehen ist, mit der Stimulationseinheit verbunden ist, wobei der zweite Sicherheitsmodus (240) zu einem zweiten Sicherheitsbetrieb des implantierbaren Systems führt, bei dem das implantierbare System bestimmte Anforderungen für die Stimulation eines His-Bündels erfüllen kann.

## Revendications

1. Système implantable destiné à stimuler un cœur humain ou un cœur animal, comprenant un processeur, une unité de mémoire, une unité de stimulation destinée à stimuler un faisceau de His ou une autre région cardiaque d'un cœur humain ou d'un cœur animal, une unité de détection destinée à détecter un signal électrique de ce cœur, et une unité de diagnostic destinée à contrôler les paramètres fonctionnels du système implantable,
dans lequel
l'unité de mémoire comprend un programme lisible par ordinateur, qui invite le processeur à mettre en œuvre les étapes suivantes lorsque le programme est exécuté sur le processeur :
a) détecter, au moyen de l'unité de diagnostic, si au moins un état de dysfonctionnement du système implantable est présent ;
**caractérisé par** les étapes suivantes consistant à :
b) contrôler (220) si une électrode pourvue pour stimuler un faisceau de His d'un cœur humain ou d'un cœur animal est connectée à l'unité de stimulation ; et
c) commuter un état de fonctionnement du système implantable en un mode de sécurité lorsqu'un état de dysfonctionnement a été détecté, le mode de sécurité étant choisi au moins parmi un premier mode de sécurité (230) et un second mode de sécurité (240), dans lequel
i) le premier mode de sécurité (230) est choisi lorsque l'étape consistant à contrôler (220) mise en œuvre dans l'étape b) a démontré qu'aucune électrode pourvue pour stimuler un faisceau de His d'un cœur humain ou d'un cœur animal n'est connectée à l'unité de stimulation, le premier mode de sécurité (230) résultant en un premier fonctionnement de sécurité du système implantable, dans lequel le système implantable ne répond pas à des exigences spécifiques pour la stimulation du faisceau de His ; et
ii) le second mode de sécurité (240) est choisi lorsque l'étape consistant à contrôler (220) mise en œuvre dans l'étape b) a démontré qu'une électrode pourvue pour stimuler un faisceau de His d'un cœur humain ou d'un cœur animal est connectée à l'unité de stimulation, le second mode de sécurité (240) résultant en un second fonctionnement de sécurité du système implantable dans lequel le système implantable est capable à des exigences spécifiques pour la stimulation du faisceau de His.

2. Système implantable selon la revendication 1, **caractérisé en ce que** des éléments d'information qui sont exigés pour commuter le système dans le mode de sécurité sont stockés dans une mémoire non volatile, une mémoire redondante ou une mémoire récupérable du système implantable.

3. Système implantable selon la revendication 1 ou 2, **caractérisé en ce que** l'étape consistant à contrôler (220) si une électrode pourvue pour stimuler un faisceau de His d'un cœur humain ou d'un cœur animal est connectée à l'unité de stimulation est mise en œuvre en se basant sur des informations stockées dans l'unité de mémoire.

4. Système implantable selon l'une quelconque des revendications précédentes, **caractérisé en ce que** l'unité de diagnostic est configurée pour détecter l'état de dysfonctionnement en se basant sur au moins l'un des événements suivants : une erreur de mémoire, une erreur d'exécution de logiciel, une erreur d'état de logiciel, une cyberattaque détectée, une erreur de paramètre, une activation d'un état de réinitialisation du système implantable, un drain de batterie et une interférence électromagnétique externe du système implantable.

5. Système implantable selon l'une quelconque des revendications précédentes, **caractérisé en ce que** le second fonctionnement de sécurité est **caractérisé par** au moins l'une des configurations suivantes : un réglage de stimulation de faisceau de His d'un pôle d'électrode de l'unité de stimulation à laquelle une électrode pourvue pour stimuler un faisceau de His d'un cœur humain ou d'un cœur animal est connectée ; une énergie de stimulation d'une stimulation à administrer par une électrode pourvue pour stimuler un faisceau de His d'un cœur humain ou d'un cœur animal ; une sensibilité de détection de l'unité de détection destinée à détecter des signaux spécifiques au faisceau de His ; un mode de fonctionnement de stimulation du système implantable ; une fréquence de stimulation d'une stimulation à administrer par une électrode pourvue pour stimuler un faisceau de His d'un cœur humain ou d'un cœur animal ; et un réglage de stimulation de faisceau de His d'un second pôle d'électrode de l'unité de stimulation à laquelle une seconde électrode destinée à stimuler un cœur humain ou un cœur animal est connectée.

6. Système implantable selon l'une quelconque des revendications précédentes, **caractérisé en ce que** le programme invite le processeur à en outre mettre en œuvre l'étape suivante consistant à :
a) commuter un état de fonctionnement du système implantable en un mode de protection lorsque le mode de protection a été activé par un signal externe, le mode de protection étant choisi au moins parmi un premier et un second mode de protection, dans lequel
i) le premier mode de protection est choisi lorsque l'étape consistant à contrôler (220) mise en œuvre dans l'étape b) a démontré qu'aucune électrode pourvue pour stimuler un faisceau de His d'un cœur humain ou d'un cœur animal n'est connectée à l'unité de stimulation, le premier mode de protection résultant en un premier fonctionnement de protection du système implantable dans lequel le système implantable ne répond pas à des exigences spécifiques pour la stimulation du faisceau de His ; et
ii) le second mode de protection est choisi lorsque l'étape consistant à contrôler mise en œuvre dans l'étape b) a démontré qu'une électrode pourvue pour stimuler un faisceau de His d'un cœur humain ou d'un cœur animal est connectée à l'unité de stimulation, le second mode de protection résultant en un second fonctionnement de protection du système implantable, dans lequel le système implantable est capable de répondre à des exigences spécifiques pour la stimulation du faisceau de His.

7. Système implantable selon l'une quelconque des revendications précédentes, **caractérisé en ce que** le second fonctionnement de sécurité est **caractérisé par** au moins l'une des configurations suivantes : un réglage de stimulation de faisceau de His d'un pôle d'électrode de l'unité de stimulation à laquelle une électrode pourvue pour stimuler un faisceau de His d'un cœur humain ou d'un cœur animal est connectée ; une énergie de stimulation d'une stimulation à administrer par une électrode pourvue pour stimuler un faisceau de His d'un cœur humain ou d'un cœur animal ; une sensibilité de détection de l'unité de détection destinée à détecter des signaux spécifiques au faisceau de His ; un mode de fonctionnement de stimulation du système implantable ; une fréquence de stimulation d'une stimulation à administrer par une électrode pourvue pour stimuler un faisceau de His d'un cœur humain ou d'un cœur animal ; et un réglage de stimulation de faisceau de His d'un pôle d'électrode supplémentaire de l'unité de stimulation à laquelle une électrode pourvue pour stimuler un faisceau de His d'un cœur humain ou d'un cœur animal est connectée.

8. Système implantable selon l'une quelconque des revendications précédentes, **caractérisé en ce que** le programme invite le processeur à établir ou adapter individuellement la configuration du second fonctionnement de sécurité en tant que fonction de paramètres de programmation réglables.

9. Système implantable selon l'une quelconque des revendications 1 à 7, **caractérisé en ce que** le programme invite le processeur à déterminer automatiquement la configuration du second fonctionnement de sécurité en tant que fonction d'éléments d'information stockés dans le système implantable.

10. Système implantable selon l'une quelconque des revendications précédentes, **caractérisé en ce que** le système implantable comprend une unité de transmission à distance de données, le programme invitant le processeur à transmettre une activation du mode de sécurité à un système de surveillance à distance au moyen de l'unité de transmission de données à distance.

11. Procédé destiné à commander le fonctionnement d'un système implantable destiné à stimuler un cœur humain ou un cœur animal selon l'une quelconque des revendications précédentes, dans lequel le procédé comprend les étapes suivantes consistant à :
a) détecter, au moyen de l'unité de diagnostic destiné à contrôler les paramètres fonctionnels du système implantable, si au moins un état de dysfonctionnement du système implantable est présent ;
b) contrôler (220) si une électrode pourvue pour stimuler un faisceau de His d'un cœur humain ou d'un cœur animal est connectée à l'unité de stimulation destinée à stimuler un faisceau de His ou une région cardiaque similaire d'un cœur humain ou d'un cœur animal ; et
c) commuter un état de fonctionnement du système implantable en un mode de sécurité lorsqu'un état de dysfonctionnement a été détecté, le mode de sécurité étant choisi au moins parmi un premier mode de sécurité (230) et un second mode de sécurité (240), dans lequel
i) le premier mode de sécurité (230) est choisi lorsque l'étape consistant à contrôler (220) mise en œuvre dans l'étape b) a démontré qu'aucune électrode pourvue pour stimuler un faisceau de His d'un cœur humain ou d'un cœur animal n'est connectée à l'unité de stimulation, le premier mode de sécurité (230) résultant en un premier fonctionnement de sécurité du système implantable, dans lequel le système implantable ne répond pas à des exigences spécifiques pour la stimulation du faisceau de His ; et
ii) le second mode de sécurité (240) est choisi lorsque l'étape consistant à contrôler (220) mise en œuvre dans l'étape b) a démontré qu'une électrode pourvue pour stimuler un faisceau de His d'un cœur humain ou d'un cœur animal est connectée à l'unité de stimulation, le second mode de sécurité (240) résultant en un second fonctionnement de sécurité du système implantable dans lequel le système implantable est capable à des exigences spécifiques pour la stimulation du faisceau de His.

12. Produit de programme informatique incluant un code lisible par ordinateur, qui invite le processeur du système implantable selon l'une quelconque des revendications 1 à 10 à mettre en œuvre les étapes suivantes lorsque le code est exécuté sur le processeur :
a) détecter, au moyen de l'unité de diagnostic destinée à contrôler les paramètres fonctionnels du système implantable destiné à stimuler un cœur humain ou un cœur animal, si au moins un état de dysfonctionnement du système implantable est présent ;
b) contrôler (220) si une électrode pourvue pour stimuler un faisceau de His d'un cœur humain ou d'un cœur animal est connectée à l'unité de stimulation destinée à stimuler un faisceau de His ou une région cardiaque similaire d'un cœur humain ou d'un cœur animal ; et
c) commuter un état de fonctionnement du système implantable en un mode de sécurité lorsqu'un état de dysfonctionnement a été détecté, le mode de sécurité étant choisi au moins parmi un premier mode de sécurité (230) et un second mode de sécurité (240), dans lequel
i) le premier mode de sécurité (230) est choisi lorsque l'étape consistant à contrôler (220) mise en œuvre dans l'étape b) a démontré qu'aucune électrode pourvue pour stimuler un faisceau de His d'un cœur humain ou d'un cœur animal n'est connectée à l'unité de stimulation, le premier mode de sécurité (230) résultant en un premier fonctionnement de sécurité du système implantable, dans lequel le système implantable ne répond pas à des exigences spécifiques pour la stimulation du faisceau de His ; et
ii) le second mode de sécurité (240) est choisi lorsque l'étape consistant à contrôler (220) mise en œuvre dans l'étape b) a démontré qu'une électrode pourvue pour stimuler un faisceau de His d'un cœur humain ou d'un cœur animal est connectée à l'unité de stimulation, le second mode de sécurité (240) résultant en un second fonctionnement de sécurité du système implantable dans lequel le système implantable est capable à des exigences spécifiques pour la stimulation du faisceau de His.
